# EUROPÄISCHE PATENTSCHRIFT

(11) **EP 1 705 485 B1**
(45) Veröffentlichungstag und Bekanntmachung des Hinweises auf die Patenterteilung: **20.08.2008**
(21) Anmeldenummer: 05006451.8
(22) Anmeldetag: 23.03.2005
(51) Int. Cl.: G01N 33/542, G01N 33/58, G01N 33/66

(54) **Verfahren zur Bestimmung der Glucosekonzentration durch Fluoreszenzpolarisation**
Procedure for the determination of the glucose concentration by fluorescence polarization
Procédure de disposition de la concentration de glucose par de polarisation de la fluorescence

(43) Veröffentlichungstag der Anmeldung: 27.09.2006
(73) Patentinhaber: Roche Diagnostics GmbH, 68305 Mannheim (DE); F. Hoffmann-La Roche AG, 4070 Basel (CH)
(72) Erfinder: Hoenes, Joachim, Dr., 64673 Zwingenberg (DE); von der Eltz, Herbert, Dr., 82362 Weilheim (DE)
(74) Vertreter: Weiss, Wolfgang

(56) Entgegenhaltungen:
- WO-A-02/03855
- US-A- 5 660 991
- BEYER U ET AL: "Recording of subcutaneous glucose dynamics by a viscometric affinity sensor" DIABETOLOGIA, BERLIN, DE, 2001, Seiten 416-423, XP002321003 ISSN: 0012-186X

## Beschreibung

Die Erfindung betrifft ein implantierbares System, welches in einem Verfahren zum *in vivo* Glucose-Monitoring verwendet werden kann bei dem die Glucosekonzentration mittels Fluoreszenzpolarisation durch Detektion der - durch die sich ändernde Glucosekonzentration bedingte - Viskositätsänderung einer Mischung, die ein Glucose-bindendes Lektin, wie beispielsweise Concanavalin A, Dextran, eine fluoreszierende Sonde und Glucose enthält, gemessen wird.

Die Fluoreszenzpolarisation ist eine bekannte, leistungsfähige Methode zur schnellen und homogenen Analyse von molekularen Wechselwirkungen in biologischen und chemischen Systemen, die erstmals von Perrin beschrieben wurde (Perrin, M.F., J. Phys. Radium, 7 (1926), 390-401; Jolley, M.E.; J. Anal. Toxicol. 5 (1981), 236).

Die Prinzipien der Fluoreszenzpolarisation basieren auf der Anregung eines fluoreszierenden Moleküls, dem Fluorophor, mit polarisiertem Licht. Diese führt zur Emission von Photonen in die Ebene, die parallel und senkrecht zur Anregungsebene ist, und gibt Informationen über die lokale Umgebung des Fluorophors.

Durch Messung der Drehung der Polarisationsebene des ursprünglich eingestrahlten polarisierten Lichts kann die Rotation von Fluorophoren in Lösung beobachtet werden.

Der Polarisationswert liegt bei gelösten, statistisch orientierten Molekülen zwischen 0 und 0,5 in Abhängigkeit von der Rotationsgeschwindigkeit der Moleküle während der Fluoreszenzlebensdauer des angeregten Zustands. Ist die Rotation schnell im Vergleich zur Fluoreszenzlebensdauer, ist die Polarisation der emittierten Strahlung nahe Null. Ist die Rotation dagegen langsam gegenüber der Fluoreszenzlebensdauer, so ist die Polarisation im emittierten Licht hoch. Kleine Moleküle rotieren schnell und haben daher einen kleineren Polarisationswert. Umgekehrt haben größere Moleküle einen größeren Polarisationswert aufgrund ihrer langsamen Rotation.

Ein Vorteil der Fluoreszenzpolarisation liegt darin, dass durch Bezug der verschiedenen polarisierten Emissions-Anteile aufeinander Intensitätsschwankungen im optischen System und in der Probelösung automatisch eliminiert werden. Somit treten bei dieser Methode in der Regel keine Fehler durch das optische System oder durch inkonsistente, z.B. verschieden stark absorbierende Proben, auf.

Die beobachtete Rotation hängt von der Rotationsrelaxationszeit ab und wird lediglich durch die Temperatur, die Viskosität und das Molekulargewicht des Fluorophors beeinflusst. Somit ist die Fluoreszenzpolarisation eine geeignete Methode, um diese Parameter und insbesondere deren Änderung zu messen. Folglich hat die Fluoreszenzpolarisation in der Diagnostik eine hohe Bedeutung.

In US 6,596,546 B1 wird die Verwendung der statischen und dynamischen Fluoreszenzpolarisation zum diagnostischen Nachweis von Komplexbildungsreaktionen beschrieben. Hierbei wird ein Antigen, das mit einem Fluorophor markiert ist und in Lösung vorliegt, mit einen Antikörper, der in einer verdünnten Serumprobe enthalten ist, umgesetzt, um einen Immunkomplex zu bilden. Der Immunkomplex wird dann durch die Änderung der Fluoreszenzpolarisation detektiert.

Nasir und Jolley (Nasir, M.S.; Jolley, M.E.; Combinatorial Chemistry & High Throughput Screening, 2 (1999), 177-190, Bentham Science Publishers B.V.) beschreiben die Anwendung der Fluoreszenzpolarisation als analytisches Werkzeug für Immunoassays, z.B. zum Nachweis von Serumproteinen, Antikörpern und Hormonen, zum Nachweis von Toxinen in Saatgut und bei der Drogendetektion.

Bei derartigen Nachweisen verwendet man im Allgemeinen fluoreszenzmarkierte Antigene oder Antikörper, deren Polarisation gemessen wird. Bei deren Reaktion mit einem spezifischen Antikörper oder Antigen, d.h. nach Bildung eines Antigen-Antikörper-Komplexes, die zur Erhöhung des effektiven Molekulargewichts führen, resultiert eine Erhöhung der Fluoreszenzpolarisation.

Die Anwendung der Fluoreszenzpolarisation als Werkzeug zur Untersuchung von Polyelektrolytkomplexen und Hydrogelen wird von Rangarajan et al. (Rangarajan, B.; Coons, L.S.; Scranton, A.B.; Biomaterials 17 (1996), 649-661) beschrieben. Bei dieser Methode wird der Fluorophor kovalent mit dem zu untersuchenden Polymer verknüpft.

Nachteilig bei bekannten diagnostischen Methoden, in denen eine Messung der Fluoreszenzpolarisation erfolgt, ist, dass eine aufwändige Optimierung des Labels, d.h. des Fluorophors und dessen Bindung an das Antigen bzw. den Antikörper, erforderlich ist, damit die eigentliche Komplexbildung nicht beeinträchtigt wird. Des Weiteren müssen Markierungen, die mittels Fluoreszenzpolarisation Änderungen im Molekulargewicht detektieren sollen, kovalent am markierten Molekül gebunden werden, da schon geringe Relativbewegungen die Fluoreszenzpolarisation und damit das Nutzsignal verringern.

Als weitere analytische Methode, bei der Fluorophore verwendet werden, ist das Fluoreszenz-Resonanz-Energietransfer (FRET)-Verfahren bekannt. Bei dem FRET-Verfahren erfolgt die strahlungsfreie Übertragung von Photonenenergie von einem angeregten Fluorophor (dem Donor) auf einen anderen Fluorophor (den Akzeptor), wenn der Abstand zwischen beiden nicht mehr als 1-10 nm beträgt. Diese Energieübertragung erfolgt strahlungsfrei, im Wesentlichen durch eine Dipol/Dipol-Wechseiwirkung. Mittels dem FRET-Verfahren können ebenfalls z.B. molekulare Wechselwirkungen zwischen zwei Proteinpartnern oder Strukturänderungen innerhalb eines Moleküls (u.a. die Enzymaktivität oder DNA/RNA-Konformation) nachgewiesen werden (Gordon, G.W.; Berry, G. et al; J. Biophys.; 74 (1998), 2702-2713 und Xia, Z.; Liu, Y.; J. Biophys.; 81 (2001), 2395-2402).

Nachteilig bei dem FRET-Verfahren ist, dass nur bestimmte Fluorophor-Paare für das FRET-Verfahren geeignet sind, da neben anderen Voraussetzungen, wie Dipol-Orientierung und ausreichende Fluoreszenzlebensdauer, das Emissionsspektrum des Donors sich mit dem Anregungsspektrum des Akzeptors überlappen muss. Es sind zwei Markierungen erforderlich, wodurch das FRET-Verfahren noch aufwändiger ist als die Fluoreszenzpolarisation.

Aus dem Stand der Technik sind verschiedene Verfahren zum Nachweis von Glucose bekannt. Ehwald et al. (Ehwald, R.; Ballerstädt, R.; Dautzenberg, H.; Analytical Biochemistry; 234 (1) (1996), 1-8) beschreiben den Nachweis von Glucose mittels der Komplexbildungsreaktion Concanavalin A (ConA)-Dextran. Die Komplexbildung wird durch Glucose gestört, wodurch eine Veränderung der Viskosität resultiert. Die Komplexierung und ihre Änderung und somit die Konzentration der Glucose können daher mittels Viskosimetrie gemessen werden. Nachteilig bei diesem Verfahren zum Nachweis von Glucose ist, dass das viskosimetrische Verfahren technisch sehr aufwändig ist.

Eine weitere Methode zur Bestimmung von Glucose basiert auf dem FRET-Verfahren, das McCartney et al. beschreiben (McCartney, L. et al.; Analytical Biochemistry; 292 (2), (2001), 216-221). Sowohl Concanavalin A als auch Dextran können mit einem Fluorophor markiert werden. Die Markierung bewirkt, dass bei Komplexbildung im Komplex eine effektive Energieübertragung vom ersten auf den zweiten Fluorophor (FRET) erfolgt. Die Zugabe von Glucose führt zur Auflösung des Komplexes, wodurch die Intensität der Strahlung aus dem zweiten Fluorophor sinkt. Ein großer Nachteil des Nachweises von Glucose mit dem FRET-Verfahren ist, dass sowohl Concanavalin A als auch Dextran mit einem Fluorophor markiert werden müssen. Zudem müssen die entsprechenden Fluorophore geeignete Fluoreszenzeigenschaften aufweisen. Auch die Reabsorption der vom ersten Fluorophor ausgesandten Strahlung durch den zweiten Fluorophor verringert das Nutzsignal, da sie auch ohne Komplexierung erfolgt.

Des Weiteren sind verschiedene Verfahren zum *in vivo* und *in vitro* kontinuierlichen Glucose-Monitoring bekannt:
- Mikrodialyse und enzymatischer Nachweis außerhalb des Körpers;
- Viskosimetrie mit Concanavalin A/Dextran (GlucOnline^{®});
- Elektrochemische Sensoren mit enzymatischer Umsetzung der Glucose im Körper (Minimed^{®});
- Langzeit-Sensoren, die z.B. auf Basis von markiertem Concanavalin A und Dextran nach der FRET-Methode arbeiten.

Es ist jedoch kein Verfahren oder System bekannt, bei dem ein *in vivo* Glucose-Monitoring zur Bestimmung der Glucosekonzentration oder eine *in vitro* Bestimmung der Glucosekonzentration mittels Fluoreszenzpolarisation durchgeführt werden können.

Eine Aufgabe der Erfindung war es daher, die Nachteile des Standes der Technik zumindest teilweise zu überwinden und ein System, welches in einem einfachen und genauen Verfahren zum *in vivo* Glucose-Monitoring mittels Fluoreszenzpolarisation verwendet werden kann, bereitzustellen.

Diese Aufgabe wird gelöst durch ein technisch einfaches System zum *in vivo* Glucose-Monitoring, bei dem die Bildung des Komplexes aus einem Glucose-bindenden Lektin und Dextran, insbesondere aus Concanavalin A und Dextran, nicht durch deren Markierung mit einem Fluorophor gestört wird.

Es wurde nun ein System zum *in vivo* Glucose-Monitoring gefunden, welches die folgenden Bestandteile umfasst:
(a) einen Behälter enthaltend ein Glucose-bindendes Lektin, vorzugsweise Concanavalin A, Dextran und eine Sonde bestehend aus einem Fluorophor und einem Träger und
(b) einen Sensor zur Messung der Fluoreszenzpolarisation.

Das oben genannte System ist geeignet zur Verwendung in einem Verfahren zum *in vivo* Glucose-Monitoring, welches das Implantieren eines für Glucose permeablen Behälters mit einem Innenraum, der eine Mischung von einem Glucose-bindenden Lektin, vorzugsweise Concanavalin A, Dextran und eine Sonde bestehend aus einem Fluorophor und einem Träger enthält, und einem Sensor zur Messung der Fluoreszenzpolarisation, in den Körper eines Individuums und das Messen der Glucosekonzentration durch Detektion einer Viskositätsänderung im Innenraum des Behälters mittels Fluoreszenzpolarisation umfasst.

Vorzugsweise umfasst das Verfahren die folgenden Schritte:
(a) Befüllen eines Behälters mit einer Mischung enthaltend ein Glucose-bindendes Lektin, vorzugsweise Concanavalin A, Dextran und eine Sonde bestehend aus einem Fluorophor und einem Träger,
(b) Verschließen des Behälters,
(c) Verbinden des Behälters mit einem Sensor, vorzugsweise einem Lichtleiter für Ein- und Ausstrahlung,
(d) Implantieren des so erhaltenen Systems unter die Haut eines Individuums und
(e) Messen der Glucosekonzentration durch Detektion der Viskositätsänderung mittels Fluoreszenzpolarisation.

Der bei dem erfindungsgemäßen Verfahren verwendete Behälter enthält eine Mischung eines Lektin-Dextran-Komplexes, vorzugsweise eines Concanavalin A-Dextran-Komplexes, und einer Sonde. Der Polarisationswert dieser Mischung wird durch den Sensor mittels der Sonde, die einen Fluorophor enthält, gemessen. Nach Implantieren des Behälters in den Körper eines Individuums diffundiert Glucose aus Körperflüssigkeiten, insbesondere aus Blut oder durch das Cytoplasma, in den Behälter. Glucose beeinflusst die Lektin-Dextran-Komplexbildung, vorzugsweise die Concanavalin A-Dextran-Komplexbildung. Dadurch resultiert eine Viskositätsänderung der in dem Behälter enthaltenen Mischung. Diese Viskositätsänderung bewirkt eine Änderung der Fluoreszenzpolarisation des in der Mischung enthaltenen Fluorophors, so dass die Änderung der Glucosekonzentration durch Messung des Polarisationswertes bestimmt werden kann. Vorzugsweise wird eine kontinuierliche Bestimmung der Glucosekonzentration durchgeführt.

Das Verfahren kann zum *in vivo* Monitoring von D- und L-Glucose verwendet werden. Vorzugsweise wird es zum *in vivo* Glucose-Monitoring von D-Glucose verwendet.

Das Verfahren unterscheidet sich somit von den aus dem Stand der Technik bekannten Verfahren dadurch, dass weder das Glucose-bindende Lektin noch Dextran mit einem Fluorophor markiert werden müssen. Des Weiteren wird keine Molekulargewichtsänderung, sondern die Viskositätsänderung der Mischung detektiert.

Bei dem Verfahren erfolgt keine Störung der Bildung des Komplexes aus dem Glucose-bindenden Lektin und Dextran, insbesondere aus Concanavalin A und Dextran, durch die Sonde. Andere Stoffe stören nur dann, wenn sie wie Glucose an das Lektin, insbesondere an Concanavalin A binden. Solche Stoffe kommen im menschlichen Körper normalerweise nur in geringen Konzentrationen vor.

Ein weiterer Vorteil des Verfahrens ist, dass die Sonde im Hinblick auf einfache Geräte ausgewählt werden kann. Des Weiteren ermöglicht das Verfahren die Verwendung - im Vergleich zum FRET-Verfahren und zur Viskosimetrie - einfacherer Geräte; so kann bei dem erfindungsgemäßen Verfahren z.B. auf die gesamte Fluidik (Pumpe, Schläuche) verzichtet werden.

Ein weiterer Vorteil des Verfahrens gegenüber herkömmlichen Enzym-basierten Verfahren zum kontinuierlichen Glucose-Monitoring ist, dass keine enzymatische Reaktion erfolgt, wodurch das erfindungsgemäße Verfahren für eine längere Anwendungsdauer geeignet ist. Des Weiteren sind auch keine Diffusionsbarrieren zur Minimierung von Störungen erforderlich, da keine unerwünschten Reaktionsprodukte, wie z.B. Wasserstoffperoxid, auftreten.

Als Behälter kommen zur Durchführung des Verfahrens flexible oder starre Behälter aus physiologisch kompatiblen Materialien infrage, die für Glucose durchlässig und für das Glucose-bindende Lektin, insbesondere für Concanavalin A, Dextran und die Sonde undurchlässig sind. Insbesondere kommen Behälter aus literaturbekannten semipermeablen Membranen wie beispielsweise Cellulose und/oder Cellulose-Derivaten, Polyamid und/oder Polyamid-Derivaten, Polysulfonen und/oder Polysulfon-Derivaten infrage. Besonders bevorzugt wird als Behälter ein Membransäckchen aus regenerierter Cellulose verwendet.

Die Ausschlussgrenze der Membran ist so zu wählen, dass das Glucose-bindende Lektin, vorzugsweise Concanavalin A, Dextran, und die fluoreszenzmarkierte Sonde nicht hindurch treten können. Bevorzugt sind Ausschlussgrenzen <100kDa, bevorzugt < 50 kDa, besonders bevorzugt 10-20 kDa.

Glucose-bindende Lektine, insbesondere Concanavalin A, und Dextran sind bekannt und käuflich erwerbbar, sie werden nach üblichen Verfahren gemischt und dabei gegebenenfalls in einem Verdünnungsmittel wie beispielsweise einer herkömmlichen Pufferlösung gelöst.

Zur Durchführung des Verfahrens kommt Dextran mit einem mittleren Molekulargewicht von 10 kDa bis 1000 kDa, bevorzugt von 20 kDa bis 500 kDa, besonders bevorzugt von 100 kDa bis 200 kDa infrage.

Zur Durchführung des Verfahrens kommen alle Glucose-bindenden Lektine, wie beispielsweise Concanavalin A (ConA), Lens culinaris-Agglutin und Pealectin-I (PSA) infrage. Besonders bevorzugt wird in dem Verfahren Concanavalin A verwendet.

Durch die Konzentration des Glucose-bindenden Lektins und Dextran, insbesondere von Concanavalin A und Dextran, kann ein bestimmter Messbereich eingestellt werden.

Die Konzentration des Glucose-bindenden Lektins, insbesondere von Concanavalin A, beträgt 0,2-5 % bevorzugt 0,5-2 %, besonders bevorzugt 0,5-1 %.

Die Konzentration von Dextran beträgt 1-20 %, bevorzugt 3-10%, besonders bevorzugt 5-8 %.

Die Sonde besteht aus einem Fluorophor und einem Träger. Der Fluorophor und der Träger sollten kovalent verbunden werden.

Als Fluorophore kommen vorzugsweise alle Fluorophore infrage, die eine Fluoreszenzlebensdauer von zwischen 10 und 1000 ns, bevorzugt > 100 ns, und eine Wellenlänge von 400 bis 800 nm haben. Die Fluorophore müssen langlebig sein, um bei der relativ langen Rotationskorrelationszeit des Trägers eine ausreichende Sensitivität für viskositätsabhängige Änderungen zu zeigen. Daher kommen vor allem Übergangsmetallkomplexe, wie beispielsweise Ruthenium-tris-bipyridyl-Derivate, infrage. Diese sind auch als Isothiocyanate oder N-Hydroxy-succinimid-ester käuflich und damit direkt zur Markierung des Trägers einsetzbar. Besonders bevorzugt wird Bis-(bipyridin)-5-(isothiocyanato-phenanthrolin)-Ru(PF₆)₂ verwendet.

Als Träger kommen physiologisch verträgliche Polymere, wie beispielsweise organische Polymere, z.B. Polyethylen oder Polyethylen-Copolymere und Biopolymere, wie Polypeptide und Proteine, wie beispielsweise Serumalbumin, infrage. Besonders bevorzugt werden Proteine verwendet. Zur Durchführung des erfindungsgemäßen Verfahrens werden vorzugsweise Träger verwendet, die unter den Verfahrensbedingungen keine oder keine signifikante Bindung an das Glucose-bindende Lektin, insbesondere an Concanavalin A, Dextran und/oder den Concanavalin A/Dextran-Komplex aufweisen. Dies ist für Proteine üblicherweise gegeben.

Das Molekulargewicht der Sonde bestehend aus dem Fluorophor und dem Träger muss so hoch sein, dass die Sonde nicht aus dem Beutel heraus diffundieren kann. Das Molekulargewicht der Sonde kann beispielsweise im Bereich von 20 kDA bis 500 kDa liegen.

Zur Durchführung des Verfahrens setzt man sehr kleine Mengen der Sonde ein. Die Menge wird von der Empfindlichkeit des Messaufbaus bestimmt.

Als Sensoren zur Messung der Fluoreszenzpolarisation kommen zur Durchführung des erfindungsgemäßen Verfahrens insbesondere optische Sensoren wie etwa Lichtleiter, beispielsweise Quarz- oder Glasfaser oder lichtleitende Polymere infrage.

Der Begriff Individuum umfasst jegliches warmblütiges Tier, insbesondere eingeschlossen Säugetiere, wie beispielsweise Menschen und nichtmenschliche Primaten wie Schimpansen und andere Affen- und Menschenaffenarten; Farmtiere wie Kühe, Schafe, Schweine, Gänse und Pferde; Haustiere wie Hunde und Katzen; Labortiere wie beispielsweise Nagetiere wie Mäuse, Ratten und Meerschweinchen und dergleichen. Erwachsene, Kinder und Neugeborene, männlich oder weiblich sollen eingeschlossen werden.

Das Implantieren des Behälters in den Körper eines Individuums erfolgt nach herkömmlichen Verfahren, beispielsweise durch Einstechen. Der Behälter wird vorzugsweise unter die Haut implantiert.

Der Behälter kann in alle geeigneten Körperteile implantiert werden, vorzugsweise in einen Arm oder unter die Bauchhaut.

Die Messung der Glucosekonzentration erfolgt intermittierend oder kontinuierlich, vorzugsweise kontinuierlich.

Ein weiterer Gegenstand der vorliegenden Erfindung ist ein System zur Bestimmung der Glucosekonzentration in einer flüssigen Probe, welches die folgenden Bestandteile umfasst:
(a) eine Mischung enthaltend ein Glucose-bindendes Lektin, vorzugsweise Concanavalin A, Dextran und eine Sonde bestehend aus einem Fluorophor und einem Träger und
(b) einen Sensor zur Messung der Fluoreszenzpolarisation.

Ein weiterer Gegenstand ist die Verwendung eines der oben genannten Systeme zur Bestimmung von Glucose in Körperflüssigkeiten, Abwasserproben oder Lebensmitteln, wobei die Körperflüssigkeiten Blut, Serum, Plasma und/oder Urin sind.

Ein weiterer Gegenstand der vorliegenden Erfindung ist eine diagnostische Zusammensetzung enthaltend ein Glucose-bindendes Lektin, vorzugsweise Concanavalin A, Dextran und eine Sonde bestehend aus einem Fluorophor und einem Träger sowie die Verwendung einer Mischung enthaltend ein Glucose-bindendes Lektin, vorzugsweise Concanavalin A, Dextran und eine Sonde bestehend aus einem Fluorophor und einem Träger zur Herstellung eines diagnostischen Mittels.

## Patentansprüche

1. System zum *in vivo* Glucose-Monitoring, welches die folgenden Bestandteile umfasst:
(a) einen Behälter enthaltend ein Glucose-bindendes Lektin, vorzugsweise Concanavalin A, Dextran und eine Sonde bestehend aus einem Fluorophor und einem Träger und
(b) einen Sensor zur Messung der Fluoreszenzpolarisation.

2. System zur Bestimmung der Glucosekonzentration in einer flüssigen Probe, welches die folgenden Bestandteile umfasst:
(a) eine Mischung enthaltend ein Glucose-bindendes Lektin, vorzugsweise Concanavalin A, Dextran und eine Sonde bestehend aus einem Fluorophor und einem Träger und
(b) einen Sensor zur Messung der Fluoreszenzpolarisation.

3. System nach Anspruch 1 oder 2 zur kontinuierlichen Bestimmung der Glucosekonzentration.

4. System nach einem der Ansprüche 1 bis 3,
**dadurch gekennzeichnet,**
**dass** als Fluorophor ein langlebiger Fluorophor mit einer Fluoreszenzlebensdauer > 100 ns verwendet wird.

5. System nach einem der Ansprüche 1 bis 4,
**dadurch gekennzeichnet,**
**dass** als Träger ein Protein verwendet wird.

6. System nach Anspruch 1,
**dadurch gekennzeichnet,**
**dass** das Molekulargewicht der Sonde bestehend aus dem Fluorophor und dem Träger so eingestellt wird, dass die Sonde nicht aus dem Behälter herausdiffundieren kann.

7. Verwendung eines Systems nach einem der Ansprüche 1 bis 6 zur Bestimmung von Glucose in einer flüssigen Probe umfassend Körperflüssigkeiten, Abwasserproben oder Lebensmittel.

8. Verwendung nach Anspruch 7, wobei die Körperflüssigkeiten Blut, Serum, Plasma und/oder Urin sind.

9. Diagnostische Zusammensetzung enthaltend ein Glucose-bindendes Lektin, vorzugsweise Concanavalin A, Dextran und eine Sonde bestehend aus einem Fluorophor und einem Träger.

10. Verwendung einer Mischung enthaltend ein Glucose-bindendes Lektin, vorzugsweise Concanavalin A, Dextran und eine Sonde bestehend aus einem Fluorophor und einem Träger zur Herstellung eines diagnostischen Mittels.

## Claims

1. A system for in vivo glucose monitoring which comprises the following constituents:
(a) a container containing a glucose-binding lectin, preferably concanavalin A, dextran and a probe consisting of a fluorophore and a carrier and
(b) a sensor for measuring fluorescence polarisation.

2. A system for determining the concentration of glucose in a liquid sample, which system comprises the following constituents:
(a) a mixture containing a glucose-binding lectin, preferably concanavalin A, dextran and a probe consisting of a fluorophore and a carrier and
(b) a sensor for measuring fluorescence polarisation.

3. A system according to claim 1 or claim 2 for the continuous determination of glucose concentration.

4. A system according to any one of claims 1 to 3,
**characterised in that**
a long-lived fluorophore with a fluorescence lifetime of > 100 ns is used as the fluorophore.

5. A system according to any one of claims 1 to 4,
**characterised in that**
a protein is used as the carrier.

6. A system according to claim 1, **characterised in that** the molecular weight of the probe consisting of the fluorophore and the support is set such that the probe cannot diffuse out of the container.

7. Use of a system according to any one of claims 1 to 6 for determining glucose in a liquid sample comprising bodily fluids, wastewater samples or foodstuffs.

8. Use according to claim 7, wherein the bodily fluids are blood, serum, plasma and/or urine.

9. A diagnostic composition containing a glucose-binding lectin, preferably concanavalin A, dextran and a probe consisting of a fluorophore and a carrier.

10. Use of a mixture containing a glucose-binding lectin, preferably concanavalin A, dextran and a probe consisting of a fluorophore and a support for producing a diagnostic agent.

## Revendications

1. Système destiné au contrôle du glucose *in vivo,* qui comprend les éléments suivants:
(a) un récipient contenant une lectine capable de se lier au glucose, de préférence de la concanavaline A, du dextrane et une sonde constituée d'un fluorophore et d'un support, et
(b) un capteur pour la mesure de la polarisation de fluorescence.

2. Système destiné à la détermination de la concentration de glucose dans un échantillon liquide, qui comprend les éléments suivants:
(a) un mélange contenant une lectine capable de se lier au glucose, de préférence de la concanavaline A, du dextrane et une sonde constituée d'un fluorophore et d'un support, et
(b) un capteur pour la mesure de la polarisation de fluorescence.

3. Système selon la revendication 1 ou 2, destiné à la détermination en continu de la concentration de glucose.

4. Système selon l'une des revendications 1 à 3, **caractérisé en ce qu'**un fluorophore de longue durée de vie ayant une durée de vie de la fluorescence > 100 ns est utilisé comme fluorophore.

5. Système selon l'une des revendications 1 à 4, **caractérisé en ce qu'**une protéine est utilisée comme support.

6. Système selon la revendication 1, **caractérisé en ce que** la masse molaire de la sonde constituée du fluorophore et du support est telle que la sonde ne puisse pas diffuser hors du récipient.

7. Utilisation d'un système selon l'une des revendications 1 à 6 pour le dosage du glucose dans un échantillon liquide englobant les liquides corporels, les échantillons d'eaux usées ou les aliments.

8. Utilisation selon la revendication 7, dans laquelle les liquides corporels sont le sang, le sérum, le plasma et/ou l'urine.

9. Composition de diagnostic contenant une lectine capable de se lier au glucose, de préférence de la concanavaline A, du dextrane et une sonde constituée d'un fluorophore et d'un support.

10. Utilisation d'un mélange contenant une lectine capable de se lier au glucose, de préférence de la concanavaline A, du dextrane et une sonde constituée d'un fluorophore et d'un support pour la préparation d'un agent de diagnostic.
